# FASCICULE DE BREVET EUROPEEN

(11) **EP 2 856 980 B1**
(45) Date de publication et mention de la délivrance du brevet: **10.03.2021**
(21) Numéro de dépôt: 14187238.2
(22) Date de dépôt: 01.10.2014
(51) Int. Cl.: A61F 2/50, A61F 2/80

(54) **Emboîture prothétique, procédé de fabrication d'une telle emboîture**
Prothesenschaft, Herstellungsverfahren eines solchen Schafts
Prosthetic socket, method for manufacturing such a socket

(30) Priorité: 01.10.2013 FR 1359511
(43) Date de publication de la demande: 08.04.2015
(73) Titulaire: Pommier Orthopedie, 91140 Villebon Sur Yvette (FR)
(72) Inventeur: Pommier, Pascal, 91300 Massy (FR)
(74) Mandataire: Cabinet Camus Lebkiri

(56) Documents cités:
- WO-A1-2014/032785
- FR-A1- 2 539 616
- FR-A1- 2 828 093
- JP-A- 2010 115 348
- US-A1- 2007 276 510
- US-A1- 2012 179 272
- US-B1- 6 231 617

## Description

### DOMAINE TECHNIQUE DE L'INVENTION

L'invention se rapporte au domaine médical, et en particulier au domaine des systèmes prothétiques destinés à remplacer un membre ou une articulation amputé(e). L'invention concerne plus particulièrement une emboîture de système prothétique, utilisée pour interfacer un membre amputé, aussi appelé moignon, de la partie inférieure du corps (jambe) et un élément modulaire de prothèse.

### ARRIERE PLAN TECHNOLOGIQUE DE L'INVENTION

Classiquement, un système prothétique pour membre inférieur comporte une prothèse constituée d'éléments modulaires (tels un genou, un tube et un pied) et une emboîture. L'emboîture est destinée à recevoir un membre inférieur amputé, et permet la suspension d'un élément modulaire. L'emboîture est effectuée sur mesure afin de s'adapter à la forme et la taille du membre inférieur amputé.

Actuellement, les emboîtures sont réalisées en résine ou en plastique rigide ou semi-rigide, et comportent une structure externe en carbone. Or l'emboîture doit être confortable, facile à chausser et avoir une bonne tenue sur le membre inférieur amputé, afin notamment de ne pas la perdre lors de la phase oscillante de la marche.

Certaines emboîtures sont placées directement en contact avec le membre inférieur amputé - ces emboîtures sont dites « à contact » - mais pour résoudre le problème précité, il est connu de faire appel à un manchon. Le manchon est destiné à être placé à l'intérieur de l'emboîture, et est le plus souvent en silicone. Le silicone est en effet un matériau souple, déformable et confortable. Le manchon facilite la mise en place du membre inférieur amputé dans l'emboîture. Il a également une fonction de confort, le manchon assurant le contact et l'amortissement du membre inférieur amputé dans l'emboîture, en réduisant les douleurs éventuelles et en améliorant la tolérance du moignon.

Par exemple, une emboîture selon le préambule de la revendication 1 est connue de FR 2 539 616, ainsi que son procédé de fabrication. D'autres emboîtures connues sont divulguées dans US 2007/0276510 et WO 2014/032785, ce dernier relevant de l'article 54(3) CBE.

Cependant les emboîtures actuelles présentent plusieurs inconvénients :
- Les variations de volume du membre inférieur amputé ne sont pas prises en compte, en particulier lorsque le membre inférieur amputé grossit : le membre inférieur amputé est alors comprimé dans l'emboîture. De telles variations de volume peuvent être dues à un développement musculaire, une prise ou une perte de poids, ou encore à un problème vasculaire. L'emboîture n'est alors plus adaptée au membre inférieur amputé et provoque l'inconfort du patient.
- La nécessité de mettre en place un manchon est une contrainte quotidienne pour le patient, de par leur éventuelle complexité. Un enfile emboîture peut également être utilisé, sollicitant fortement les membres supérieurs.
- L'emboîture est inconfortable en position assise du fait de sa rigidité, l'emboîture ne s'adaptant pas à la déformation du membre inférieur amputé.
- L'emboîture est inconfortable lorsqu'elle est utilisée pendant de longues périodes.
- Les matériaux mis en œuvre dans la conception des emboîtures à contact et l'usure des manchons obligent à un renouvellement régulier de ces dispositifs.
- La difficulté d'entretien dans de bonnes conditions d'hygiène des emboîtures à contact est un problème récurrent constaté chez les personnes amputées.
- Les rotations de l'emboîture par rapport au membre inférieur sont difficiles à contrôler, en particulier avec des systèmes de manchon à accroche distale recouvert de tissu.

### DESCRIPTION GENERALE DE L'INVENTION

L'invention propose une emboîture permettant de résoudre les inconvénients précités.

L'invention concerne donc essentiellement une emboîture pour interfacer un membre inférieur amputé et un élément modulaire, telle que définie dans la revendication 1. Différents modes de réalisation préférentiels sont définis dans les revendications dépendantes 2 à 4.

On appelle mât un élément de forme allongée, comportant une base et une extrémité, et s'étendant sensiblement axialement depuis la base vers l'extrémité. La base est située du côté distal de l'emboiture, et l'extrémité est située du côté proximal de l'emboîture. Les bases des différents mâts sont solidaires les unes des autres. En revanche, les extrémités des différents mâts ne sont pas liées, et sont ainsi aptes à s'écarter les unes des autres. On considère donc que les mâts sont aptes à s'écarter ou se rapprocher les uns des autres par écartement ou rapprochement de leurs extrémités. Naturellement, les mâts sont suffisamment larges et résistants pour que l'emboiture puisse supporter le poids du corps du patient.

La gomme de silicone étant un matériau déformable, souple et élastique, elle apporte une souplesse à l'emboîture lui permettant de s'adapter à des variations de volume du membre inférieur amputé, ainsi qu'à ses variations de formes (en position assise par exemple). Utiliser un manchon n'est alors plus nécessaire. Conjuguée à la structure rigide de mâts, la gomme de silicone confère un caractère dynamique à l'emboîture. En outre, la gomme de silicone est résistante, ce qui est primordial étant donné que l'emboîture selon l'invention doit pouvoir supporter le poids du corps du patient : la couche interne et la couche externe ne doivent pas se déchirer lorsque les mâts s'écartent les uns des autres.

De plus, au niveau de la couche interne, la gomme de silicone apporte un confort au patient. Au niveau de la couche externe, la gomme de silicone permet de donner un aspect esthétique à l'emboîture en recouvrant la structure en matériau rigide. La couche externe de gomme de silicone permet également de solidariser et créer un effet dynamique entre les différentes couches qui agiront ainsi en synergie. On note que la gomme de silicone peut être pigmentée de sorte à lui donner la couleur de la peau du patient. De plus, la gomme de silicone peut être utilisée dans toutes les duretés shore disponibles, selon les besoins. On note également que la gomme de silicone absorbe peu les substances corporelles, retient peu les odeurs, et est facile d'entretien.

De plus, le matériau rigide permet la tenue de l'emboîture. Les mâts de la structure en matériau rigide sont avantageusement répartis sensiblement uniformément sur la périphérie de l'emboîture. Les mâts sont aptes à s'écarter ou se rapprocher les uns des autres en fonction des variations de volume du membre inférieur amputé, et en fonction du mouvement effectué ou de la position du patient. Un effet ressort est obtenu, la gomme de silicone jouant le rôle d'amortisseur.

Outre les caractéristiques qui viennent d'être évoquées dans le paragraphe précédent, l'emboîture selon l'invention peut présenter une ou plusieurs caractéristiques complémentaires parmi les suivantes, considérées individuellement ou selon toutes les combinaisons techniquement possibles.

Dans un mode de réalisation non limitatif, le matériau rigide comprend du carbone. Le carbone est en effet un matériau résistant et léger, il apporte une rigidité à l'emboîture. Un matériau en fibre de verre, polyamides, kevlar (aramides), de résistance moindre, pourraient être utilisé en complément du carbone.

Dans un mode de réalisation non limitatif, la structure en matériau rigide comporte quatre mâts. Un premier mât est alors destiné à être positionné sur la face antérieure du membre inférieur amputé, un deuxième mât est destiné à être positionné sur la face postérieure du membre inférieur amputé, les deux derniers mâts sont destinés à être positionnés sur chaque partie latérale du membre inférieur amputé. Ainsi, lorsque le patient est en position assise, les mâts antéro postérieurs se rapprochent, tandis que les mâts latéraux s'écartent : le confort du patient est optimal. De plus, cette déformation évite les entrées d'air au niveau de la partie proximale de l'emboîture.

Dans un mode de réalisation non limitatif, l'emboîture comporte, au niveau de la partie distale, des moyens de fixation de l'élément modulaire solidaires de la structure en matériau rigide. Les moyens de fixation sont ainsi solidement accrochés à l'emboîture et permettent de supporter le poids de la prothèse et du patient.

L'invention concerne également un procédé de fabrication d'une emboîture pour interfacer un membre inférieur amputé et un élément modulaire, tel que défini dans la revendication 5. Différents modes de réalisation préférentiels sont définis dans les revendications dépendantes 6 à 11.

Le procédé décrit permet de réaliser l'emboîture précédemment évoquée. On note que toutes les étapes d'application d'un matériau sont réalisées dans une pièce climatisée, afin d'éviter le durcissement du matériau. Puis, les étapes d'étuves permettent le durcissement du matériau.

Outre les caractéristiques qui viennent d'être évoquées dans le paragraphe précédent, le procédé selon l'invention peut présenter une ou plusieurs caractéristiques complémentaires parmi les suivantes, considérées individuellement ou selon toutes les combinaisons techniquement possibles.

Dans un mode de réalisation non limitatif, l'étape d'application sur le positif de gomme de silicone comporte les sous-étapes suivantes :
- extruder de la gomme de silicone de sorte à former au moins une bande d'épaisseur sensiblement constante ;
- recouvrir au moins une zone du positif par ladite bande ;
- étoffer la partie distale du positif ;
- découper et/ou lisser d'éventuelles surépaisseurs résiduelles.

Les bandes sont avantageusement réalisées au moyen d'une cardeuse électrique. Au terme de l'étape d'application sur le positif de la gomme de silicone, le positif est recouvert. Avantageusement, plusieurs bandes de mêmes épaisseurs ou bien d'épaisseurs différentes sont utilisées, et placées sur le positif en fonction des zones et des besoins.

Dans un mode de réalisation non limitatif, le procédé comporte l'étape suivante, entre l'étape de rigidification et l'étape de découpe : retirer la couche rigidifiée du positif recouvert de la couche interne. Le matériau rigide peut alors être découpé au moyen d'une scie électrique.

Dans un mode de réalisation non limitatif, le procédé comporte les étapes suivantes, suite à l'étape de découpe :
- positionner la structure rigide sur le positif recouvert de la couche interne ;
- coller ladite structure rigide sur la couche interne.

Dans un mode de réalisation non limitatif, le procédé comporte les étapes suivantes, suite à l'étape d'application sur le positif de la couche interne :
- recouvrir le positif recouvert de la couche interne par un sac soluble ;
- réaliser une mise sous vide du positif recouvert de la couche interne et du sac soluble.

Dans un mode de réalisation non limitatif, le procédé comporte l'étape suivante, suite à l'étape d'application sur le positif de la couche externe :
- réaliser une mise sous vide du positif recouvert de la couche interne, de la structure rigide, et de la couche externe.

Dans un mode de réalisation non limitatif, le procédé comporte l'étape suivante :
- accrocher des moyens de fixation à la structure en matériau rigide, adaptés pour coopérer avec l'élément modulaire.

L'invention et ses différentes applications seront mieux comprises à la lecture de la description qui suit et à l'examen des figures qui l'accompagnent.

### BREVE DESCRIPTION DES FIGURES

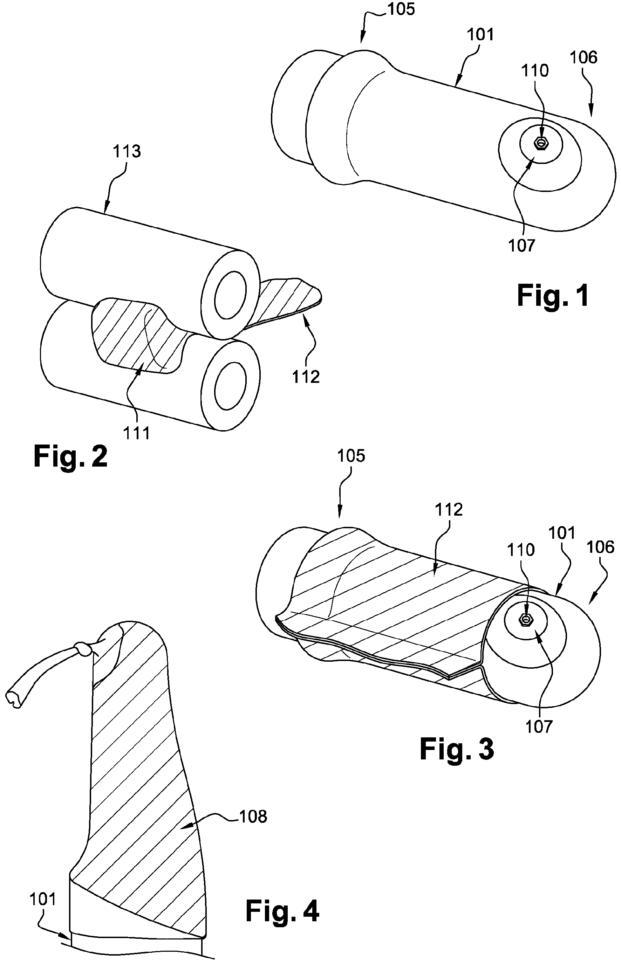

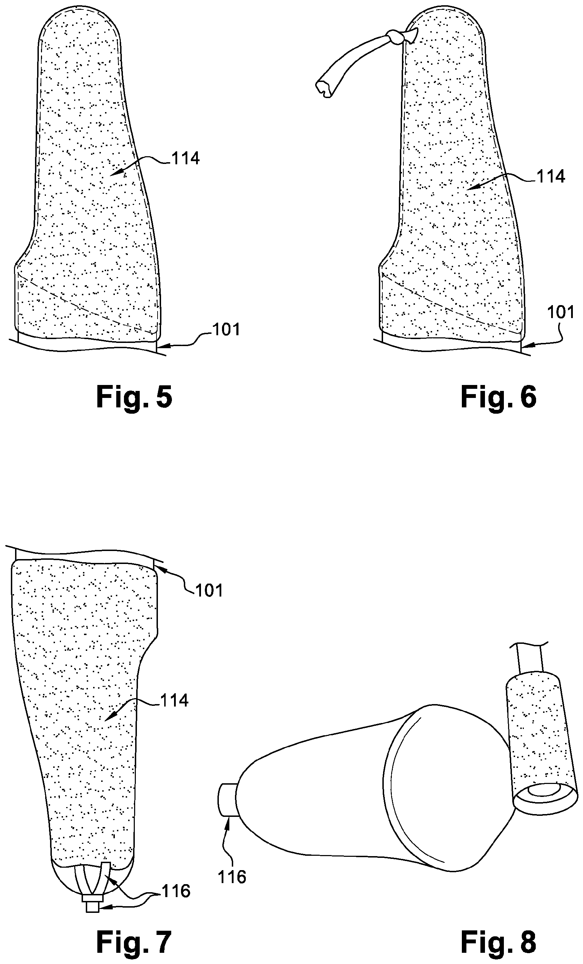

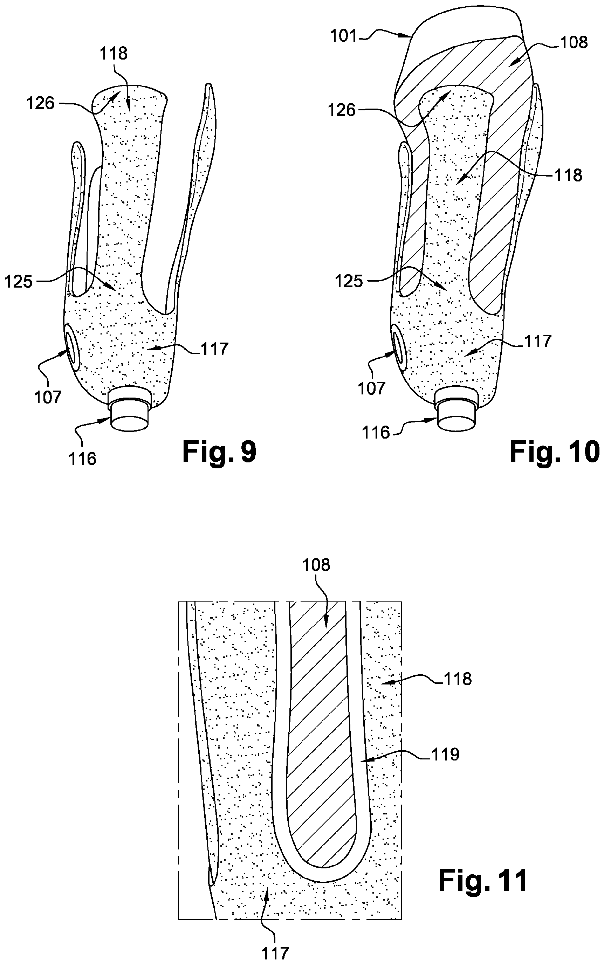

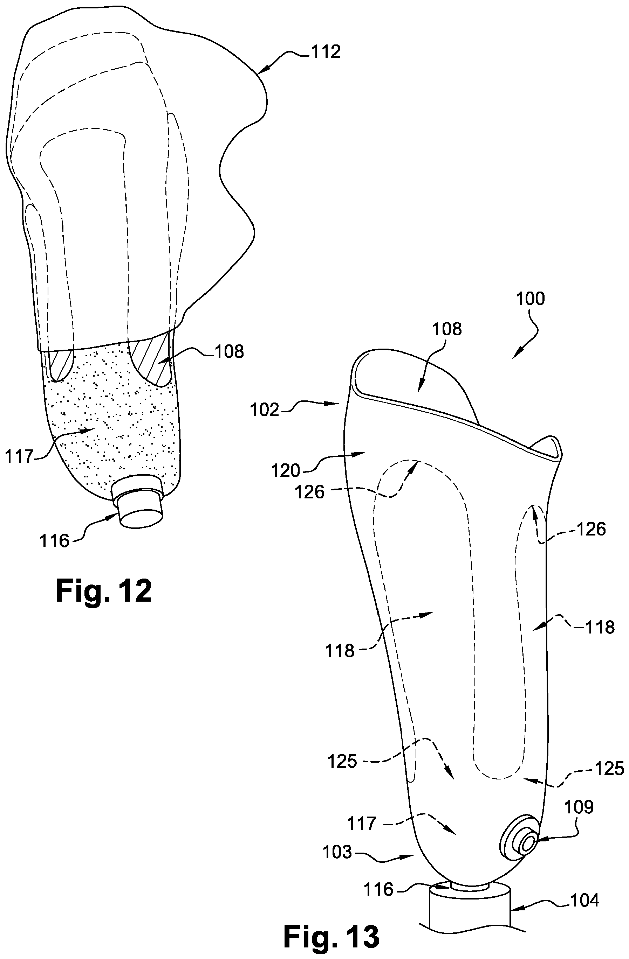

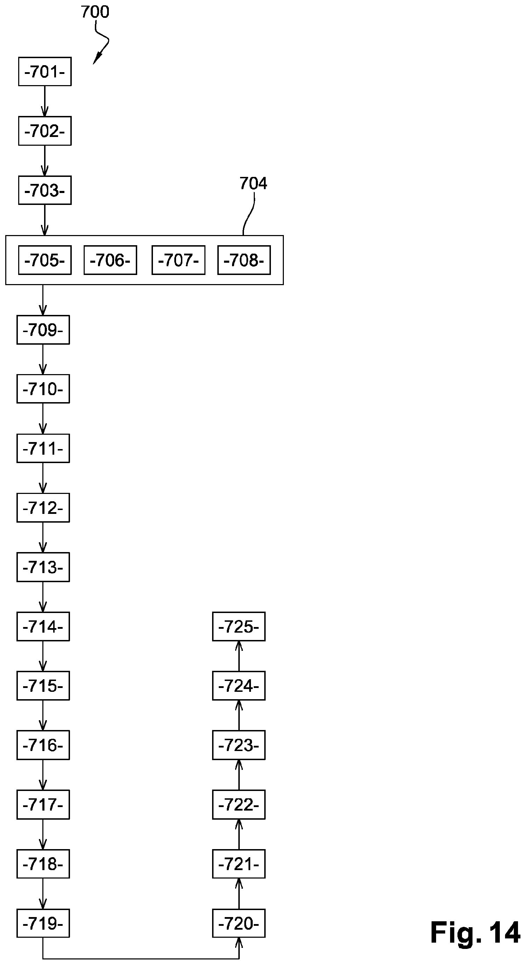

Les figures ne sont présentées qu'à titre indicatif et nullement limitatif de l'invention. Les figures montrent :
- A la figure 1, une représentation schématique d'une étape de mise en place d'un gabarit pour valve de dépressurisation, sur une partie distale d'un positif représentant un membre inférieur amputé, ladite étape étant réalisée lors d'un procédé de fabrication d'une emboîture selon un mode de réalisation non limitatif de l'invention ;
- A la figure 2, une représentation schématique d'une étape d'extrusion de gomme de silicone ;
- A la figure 3, une représentation schématique d'une étape d'application de gomme de silicone extrudé, sur le positif;
- A la figure 4, une représentation schématique d'une étape de mise sous vide du positif recouvert de gomme de silicone ;
- A la figure 5, une représentation schématique d'une étape d'application de carbone sur le positif recouvert de gomme de silicone ;
- A la figure 6, une représentation schématique d'une étape de mise sous vide du positif recouvert de gomme de silicone et de carbone ;
- A la figure 7, une représentation schématique d'une étape d'accroche de moyens de fixation pour élément modulaire, sur la couche de carbone ;
- A la figure 8, une représentation schématique d'une étape de ponçage de la couche de carbone ;
- A la figure 9, une représentation schématique d'une étape de découpe de la couche de carbone, pour former une structure rigide comportant des mâts ;
- A la figure 10, une représentation schématique d'une étape de positionnement de la structure rigide sur le positif recouvert de gomme de silicone ;
- A la figure 11, une représentation schématique d'une étape de collage de la structure rigide sur le positif recouvert de gomme de silicone ;
- A la figure 12, une représentation schématique d'une étape d'application de gomme de silicone sur le positif recouvert de gomme de silicone et de la structure rigide ;
- A la figure 13, une représentation schématique d'une emboîture selon un mode de réalisation non limitatif de l'invention ;
- A la figure 14, un diagramme représentatif des étapes du procédé.

### DESCRIPTION DETAILLEE D'AU MOINS UN MODE DE REALISATION DE L'INVENTION

Sauf précision contraire, un même élément apparaissant sur des figures différentes présente une référence unique.

L'invention concerne un procédé de fabrication d'une emboîture, ainsi que l'emboîture obtenue par le procédé. L'emboîture fabriquée est réalisée sur mesure, à partir d'un positif représentant un membre inférieur amputé d'un patient. Des moyens de fixation solidaires de l'emboîture permettent d'y fixer un élément modulaire d'une prothèse, ladite prothèse étant constituée d'au moins un élément modulaire.

Afin de limiter les risques de variation de volume du membre inférieur amputé suite au port de l'emboîture fabriquée, plusieurs prototypes d'emboîtures (en matière plastique) sont essayés pendant une ou plusieurs semaines par le patient, avant de mettre en œuvre le procédé selon l'invention qui permet de fabriquer l'emboîture finale. En effet, suite au port d'un système prothétique, le membre inférieur amputé peut se muscler et ainsi changer de volume. Il est donc important de partir d'un positif représentant le membre inférieur amputé après une ou plusieurs semaines d'utilisation d'une prothèse.

Dans un mode de réalisation non limitatif, le procédé 700 de fabrication d'une emboîture 100 (représentée à la figure 13) comporte les étapes suivantes, en référence à la figure 14 :
- Selon une étape 701 : réaliser un positif 101, visible à la figure 1, représentant le membre inférieur amputé. L'emboîture 100 est réalisée par pose de couches successives de matière sur le positif 101, qui sont rigidifiées puis démoulées du positif 101. L'emboîture 100 obtenue comporte une partie proximale 102 et une partie distale 103. La partie distale 103, fermée, permet la fixation d'un élément modulaire 104. La partie proximale 102, ouverte, permet l'insertion du membre inférieur amputé. Par analogie, on dira que le positif 101 comporte également une zone proximale 105 et une zone distale 106 : la zone distale 106 représente l'extrémité amputée ; la zone proximale 105 est l'autre extrémité. Le positif 101 est réalisé à partir d'un négatif pris par bande plâtrée ou par un dispositif informatique sur le membre inférieur amputé du patient. Le positif 101 est avantageusement réalisé en plâtre.
- Selon une étape 702 : étuver le positif 101. En effet, le positif plâtré 101 doit être parfaitement sec pour ne pas compromettre l'application d'une couche interne de gomme de silicone 108 (représentée à la figure 4) telle qu'évoquée ci-après. Le positif en plâtre 101 est étuvé dans un four spécifique jusqu'à séchage complet.
- Selon une étape 703 : en référence à la figure 1, mettre en place 703 un gabarit 107 comportant une vis de fixation 110, sur la partie distale 106 du positif 101. Le gabarit permet la fixation ultérieure d'une valve à dépressurisation 109 (représentée à la figure 13) par un système vis/écrou. Le pourtour du gabarit 107 dispose d'une dureté supérieure à la dureté de la couche interne de gomme de silicone 108 évoquée ci-après, pour renforcer la tenue de la valve 109, afin d'assurer le bon fonctionnement du dispositif. Dans une étape ultérieure du procédé, la valve 109 siègera à l'emplacement du gabarit 107.
- Selon une étape 704 : appliquer une couche de gomme de silicone 108 sur le positif 101, de sorte à le recouvrir d'une couche appelée « couche interne 108 ». On note que cette étape est réalisée dans une pièce climatisée, dont la température ambiante est inférieure à la température de durcissement de la gomme de silicone. En effet, il importe que la gomme de silicone reste malléable avant le moment désiré. Cette étape 704 comporte les sous-étapes suivantes :
   ∘ Selon une sous-étape 705 : en référence à la figure 2, extruder au moins un bloc de gomme de silicone 111 de sorte à former des bandes 112 d'épaisseur sensiblement constantes, les bandes 112 pouvant être de différentes épaisseurs les unes par rapport aux autres. La gomme de silicone extrudée est avantageusement une gomme silicone poly additive à choix de dureté multiple s'exprimant en shore (10 shore étant sa dureté minimale, 90 shore étant sa dureté maximale). Dans un mode de réalisation non limitatif, deux parties égales de la gomme silicone bi-composantes sont mélangées dans une cardeuse électrique 113 afin de créer un corps synergétique.
   ∘ Selon une sous-étape 706 : en référence à la figure 3, entourer le positif 101 d'une bande 112 de gomme de silicone. La bande 112 est travaillée avec divers outils afin d'épouser parfaitement le positif 101 pour que celui-ci dispose d'un état de surface régulier. Avantageusement, plusieurs bandes 112 d'épaisseurs différentes sont utilisées, et placées sur différentes zones du positif 101 en fonction des besoins.
   ∘ Selon une sous-étape 707 : étoffer la partie distale 106 du positif 101, au moyen d'une bande 112 de gomme de silicone. On note que seul le pourtour du gabarit 107 est recouvert, afin de permettre la mise en place ultérieure de la valve 109 tout en assurant l'étanchéité du dispositif.
   ∘ Selon une sous-étape 708 : découper et/ou lisser d'éventuelles surépaisseurs résiduelles.

Au terme de l'étape 704 d'application sur le positif 101 de la gomme de silicone, le positif 101 est recouvert.
- Selon une étape 709 : en référence à la figure 4, réaliser une mise sous vide du positif 101 recouvert de la couche interne 108. La mise sous vide permet d'assurer un bon contact de la gomme de silicone 108 sur le positif 101 en supprimant l'air résiduel entre le positif 101 et la couche interne de gomme de silicone 108.
- Selon une étape 710 : étuver le positif 101 recouvert de la couche interne 108, afin de durcir la gomme de silicone.
- Selon une étape 711 : recouvrir le positif 101 recouvert de la couche interne 108 par un sac soluble, de type sac PVA. Ceci permet d'isoler la couche interne 108 de la résine carbone 114 qui sera laminée par-dessus, afin de séparer facilement le carbone 114 de la gomme de silicone 108 par la suite. On note qu'on peut humidifier le sac soluble pour l'assouplir.
- Selon une étape 712 : en référence à la figure 5, appliquer sur le positif 101 recouvert de la couche interne 108, une fibre de carbone 114 laminée ou pré-imprégnée, de sorte à le recouvrir. La fibre de carbone 114 est imprégnée de résine. On note qu'une fibre de carbone pré-imprégnée est plus légère qu'une fibre de carbone laminée mais demande plus de temps de mise en œuvre.
- Selon une étape 713 : en référence à la figure 6, réaliser une mise sous vide du positif 101 recouvert de la couche interne 108 et de la fibre de carbone 114. En effet, pour réaliser la stratification ou le pré-imprégné, il est nécessaire de mettre sous vide les fibres de carbone et la résine pour réaliser une bonne imprégnation des tissus, afin de créer un matériau composite exempt d'imperfections.
- Selon une étape 714 : durcir la résine afin de former une couche de carbone rigide 114. Le durcissement est réalisé par catalysation pour la lamination classique, et par thermodurcissement pour le carbone pré-imprégné.
- Selon une étape 715 : retirer la couche de carbone rigidifiée 114 du positif 101 recouvert de la couche interne 108. Le sac soluble facilite le démoulage de la couche de carbone rigide 114.
- Selon une étape 716 : en référence à la figure 7, accrocher des moyens de fixation 116 de l'élément modulaire 104 à la couche rigide 114. Les moyens de fixation 116 (l'ancrage) sont collés à la couche rigide 114 puis solidarisés à celle-ci grâce à une seconde lamination de résine et carbone recouvrant une partie des moyens de fixation 116. Les alignements de l'emboîture 100 et de l'élément modulaire 104, prédéfinis lors de l'essayage, sont conservés grâce à une table de transfert d'alignements.
- Selon une étape 717 : en référence à la figure 8, poncer et polir la couche rigide 114.
- Selon une étape 718 : en référence à la figure 9, découper la couche rigide 114, de sorte à former une structure en carbone 117 comportant au moins deux mâts 118, chacun des mâts 118 s'étendant depuis une base 125 située du côté de la partie distale 103 vers une extrémité 126 située du côté de la partie proximale 102. Le nombre de mâts 118, leurs emplacements et leurs dimensions dépendent de l'amputation, des besoins du patient, et des effets dynamiques désirés par le prothésiste. La configuration et la sensibilité du membre inférieur amputé, la morphologie, et le sexe du patient influencent également ces paramètres.
- Selon une étape 719 : en référence à la figure 10, positionner la structure en carbone 117 sur le positif 101 recouvert de la couche interne 108.
- Selon une étape 720 : en référence à la figure 11, coller ladite structure rigide 117 sur la couche interne 108, par exemple à l'aide d'une colle silicone 119 appliquée sur les zones de contact entre la structure rigide 117 en carbone et la couche interne 108 en gomme de silicone.
- Selon une étape 721 : en référence à la figure 12, appliquer une couche de gomme de silicone 120 sur le positif 101 recouvert de la couche interne 108 et de la structure en carbone 117, de sorte à le recouvrir d'une couche appelée « couche externe 120 ». Cette étape comprend des sous-étapes similaires à celles décrites précédemment à propos de l'application de la couche interne 108.
- Selon une étape 722 : réaliser une mise sous vide du positif 101 recouvert de la couche interne 108, de la structure en carbone 117, et de la couche externe 120, afin d'assurer de bons contacts entre les différentes couches.
- Selon une étape 723 : étuver le positif 101 recouvert de la couche interne 108, de la structure en carbone 117, et de la couche externe 120, afin de durcir la couche externe 120.
- Selon une étape 724 : démouler l'ensemble comportant la couche interne 108, la structure rigide 117, et la couche externe 120, du positif 101. Pour ce faire, le positif en plâtre 101 est cassé, par exemple à l'aide d'un marteau et d'un burin.
- Selon une étape 725 : visser la valve de dépressurisation 109 en remplacement du gabarit 107. On note que le carbone 114 a été préalablement ajouré autour du gabarit 107, et que la couche externe de gomme de silicone 120 ne le recouvre pas non plus, afin d'en permettre l'accès. La valve de dépressurisation 109 peut ainsi être positionnée et vissée. La figure 13 montre l'emboîture 100 terminée, fixée à un élément modulaire 104. On note que la couche externe 120 peut être pigmentée afin de lui donner la couleur de la peau du patient. Des détails tels des grains de beautés, taches de rousseur ou encore pilosités peuvent aussi être ajoutés.

L'emboîture selon l'invention améliore le confort quotidien du patient et présente de nombreux avantages :
- La possibilité de chaussage en direct, sans nécessité d'utiliser un manchon, facilite la mise en place de l'emboîture. Le patient oint le membre inférieur amputé de crème ou autre produit lubrifiant et enfile directement l'emboîture. Il est néanmoins possible d'utiliser un enfile-prothèse. Le chaussage en direct permet le repositionnement aisé de la prothèse, grâce à la flexibilité de l'emboîture. Cette opération devient donc plus rapide. On note que le chaussage en direct est facilité dans le cas de membres inférieurs amputés longs et toniques. L'emboîture est donc une emboîture de contacts, utilisable sans manchon, et qui apporte un confort des chairs du membre inférieur amputé grâce à sa structure interne en gomme de silicone.
- La flexibilité complète et dans tous les plans de l'emboîture la rend confortable dans de nombreuses situations. A la marche, la déformation des mâts carbone permet de réduire les contraintes d'appui et de créer une dynamique par l'énergie restituée par l'emboîture. Le patient est plus libre de ses mouvements et le membre inférieur amputé se comporte plus naturellement dans ses sollicitations musculo-squelettiques au cours de la marche. La structure en gomme de silicone qui épouse et fait corps avec le membre inférieur amputé suit les mouvements du patient en réduisant singulièrement les contraintes d'appui dans la zone périnéale. Un amortissement des chocs sur le squelette est constaté. En position assise, l'écrasement des mâts antéro postérieurs et l'écartement des mâts latéraux permet un meilleur confort et une attitude plus naturelle. En outre, cette déformation évite les entrées d'air par le collet de l'emboîture autour du membre inférieur amputé et par conséquent le déchaussage, et supprime l'effet de rotation de l'emboîture autour du membre inférieur amputé après une assise prolongée. Les mouvements de bascule du tronc vers l'avant (par exemple pour ramasser un objet au sol) sont grandement facilités en limitant les forces exercées sur le pli inguinal par l'emboîture. En effet, la gomme de silicone se recourbe pour accompagner la flexion. Le confort est également ressenti par la diminution des éventuelles douleurs dorsolombaires grâce à un meilleur équilibre du bassin, induisant une assise plus physiologique et un axe tronc/cuisse similaire des deux côtés. Différents types de surfaces sont alors mieux tolérées par le patient : chaises rigides, assises penchées, selles de vélo, etc. De manière générale, le confort est accru dans diverses activités quotidiennes et sportives. Le port de la prothèse est mieux toléré sur de longues périodes et ne nécessite pas de rechaussage.
- La liberté du travail musculaire au sein de l'emboîture renforce la tonicité du membre inférieur amputé. L'emboîture faisant corps avec celui-ci, le patient a alors une meilleure maîtrise de la prothèse. Le renforcement musculaire participe à un meilleur aspect du membre inférieur amputé qui est alors plus stable et permet une plus grande précision des mouvements lors de la marche. En outre, l'état général du patient est préservé, ce qui prolonge son autonomie à court et long terme.
- Les caractéristiques de l'emboîture fabriquée permettent, par une bonne transmission des sensations au membre inférieur amputé, une meilleure perception de l'environnement de marche. La proprio-perception est donc améliorée, ce qui favorise le contrôle de la prothèse grâce au ressenti du membre inférieur amputé. Le patient peut ainsi ressentir le toucher au travers de l'emboîture, et par-là même la nature du support lors de l'assise. Une charge (le poids d'un enfant, d'une valise...) sur les cuisses est ainsi perçue.
- Les amplitudes articulaires sont améliorées dans tous les plans de mouvements de la hanche grâce à la souplesse de l'emboîture (dans le plan frontal : les mouvements d'abduction et d'adduction ; dans le plan sagittal : les mouvements de flexion, d'extension et les rotations).
- La souplesse de l'emboîture permet la fluctuation du volume du membre inférieur amputé dans les cas de reprise de tonus musculaire, de globulisation des muscles, de prise ou de perte de poids, ou de fluctuations quotidiennes dues à divers facteurs (œdème, chaleur...). L'emboîture est donc moins sujette au renouvellement. De plus, l'utilisation de matériaux d'une grande résistance à l'usure (gomme silicone et carbone) prolonge la durée de vie de l'emboîture.
- La découpe de l'emboîture et l'adhérence de la gomme de silicone à la peau améliorent les qualités esthétiques de celles-ci. L'emboîture se fait plus discrète sous les pantalons et la fesse reprend forme. Il est par ailleurs possible de personnaliser la gomme de silicone en l'approchant au plus près de la couleur de la peau du patient et en y ajoutant divers détails (poils, grains de beauté...). L'usure des vêtements est diminuée par une suppression des contraintes de l'emboîture sur ceux-ci, notamment en position assise.
- L'emboîture fabriquée est compatible avec tous les systèmes prothétiques disponibles (pieds, genoux) ainsi que les pièces à usage aquatique.

## Revendications

1. Emboîture (100) pour interfacer un membre inférieur amputé et un élément modulaire (104), comportant :
- une partie proximale (102) permettant l'insertion du membre inférieur amputé ;
- une partie distale (103) permettant la fixation de l'élément modulaire (104), l'emboiture comportant en outre :
- une couche interne (108) déformable destinée à être en contact avec le membre inférieur amputé ;
- une couche externe (120) déformable;
- une structure en matériau rigide (117) disposée entre la couche interne (108) et la couche externe (120), comportant au moins deux mâts (118), chaque mât (118) s'étendant sensiblement axialement depuis une base (125) située du côté distal vers une extrémité (126) située du côté proximal,
**caractérisée en ce que** :
- les couches interne (108) et externe (120) déformables sont en gomme de silicone,
et **en ce que**
- ladite structure rigide (117) est collée sur la couche interne (108), la couche interne (108) étant en contact avec la couche externe (120) entre deux mâts (118) successifs, les extrémités (126) des différents mâts (118) n'étant pas liées de sorte à s'écarter ou se rapprocher les unes des autres sous l'effet d'une déformation de la couche interne (108) et de la couche externe (120).

2. Emboîture (100) selon l'une des revendications précédentes, **caractérisé en ce que** le matériau rigide comprend du carbone.

3. Emboîture (100) selon l'une des revendications précédentes, **caractérisé en ce que** la structure en matériau rigide (117) comporte quatre mâts (118).

4. Emboîture (100) selon l'une des revendications précédentes, **caractérisé en ce qu'**elle comporte, au niveau de la partie distale (106), des moyens de fixation (116) de l'élément modulaire (104) solidaires de la structure en matériau rigide (117).

5. Procédé (700) de fabrication d'une emboîture (100) pour interfacer un membre inférieur amputé et un élément modulaire (104), **caractérisé en ce qu'**il comporte les étapes suivantes :
- réaliser (701) un positif (101) représentant le membre inférieur amputé, comprenant une zone proximale (105) et une zone distale (106) ;
- appliquer (704) sur le positif (101) de la gomme de silicone, de sorte à le recouvrir d'une couche appelée couche interne (108) ;
- étuver (710) le positif (101) recouvert de la couche interne (108) ;
- appliquer (712) sur le positif (101) recouvert de la couche interne (108), une couche (114) d'un matériau apte à être rigidifié, de sorte à le recouvrir ;
- rigidifier (714) ladite couche (114) de matériau apte à être rigidifié ;
- découper (718) ladite couche rigidifiée (114), de sorte à former une structure rigide (117) comportant au moins deux mâts (118) s'étendant de la zone distale (106) vers la zone proximale (105) ;
- appliquer (721) sur le positif (101) recouvert de la couche interne (108) et de la structure rigide (117), de la gomme de silicone, de sorte à le recouvrir d'une couche appelée couche externe (120) ;
- étuver (723) le positif (101) recouvert de la couche interne (108), de la structure rigide (117), et de la couche externe (120) ;
- démouler (724) l'ensemble comportant la couche interne (108), la structure rigide (117), et la couche externe (120), du positif (101), l'emboiture ainsi démoulée comportant différents mâts (118) dont les extrémités (126) ne sont pas liées de sorte à s'écarter ou se rapprocher les unes des autres sous l'effet d'une déformation de la couche interne (108) et de la couche externe (120).

6. Procédé (700) selon la revendication précédente, **caractérisé en ce que** l'étape d'application (704) sur le positif (101) de gomme de silicone comporte :
- extruder (705) de la gomme de silicone de sorte à former au moins une bande (112) d'épaisseur sensiblement constante ;
- recouvrir (706) au moins une zone du positif (101) par ladite bande (112) ;
- étoffer (707) la partie distale (106) du positif (101) ;
- découper (708) et/ou lisser d'éventuelles surépaisseurs résiduelles.

7. Procédé (700) selon l'une des revendications 5 à 6, **caractérisé en ce qu'**il comporte l'étape suivante, entre l'étape de rigidification (714) et l'étape de découpe (718) :
- retirer (715) la couche rigidifiée (114) du positif (101) recouvert de la couche interne (108).

8. Procédé (700) selon la revendication précédente, **caractérisé en ce qu'**il comporte les étapes suivantes, suite à l'étape de découpe (718) :
- positionner (719) la structure rigide (117) sur le positif (101) recouvert de la couche interne (108) ;
- coller (720) ladite structure rigide (117) sur la couche interne (108).

9. Procédé (700) selon l'une des revendications 5 à 8, **caractérisé en ce qu'**il comporte les étapes suivantes, suite à l'étape d'application (704) sur le positif (101) de la couche interne (108) :
- réaliser (709) une mise sous vide du positif (101) recouvert de la couche interne (108) et du sac soluble
- recouvrir (711) le positif (101) recouvert de la couche interne (108) par un sac soluble.

10. Procédé (700) selon l'une des revendications 5 à 9, **caractérisé en ce qu'**il comporte l'étape suivante, suite à l'étape d'application (721) sur le positif (101) de la couche externe (120) :
- réaliser (722) une mise sous vide du positif (101) recouvert de la couche interne (108), de la structure rigide (117), et de la couche externe (120).

11. Procédé (700) selon l'une des revendications 5 à 10, **caractérisé en ce qu'**il comporte l'étape suivante :
- accrocher (716) des moyens de fixation (116) à la structure rigide (117), adaptés pour coopérer avec l'élément modulaire (104).

## Patentansprüche

1. Schaft (100) als Schnittstelle zwischen einer amputierten unteren Gliedmaße und einem modularen Element (104), aufweisend:
- einen proximalen Teil (102), der das Einsetzen der amputierten unteren Gliedmaße erlaubt;
- einen distalen Teil (103), der die Befestigung des modularen Elements (104) erlaubt,
wobei der Schaft ferner umfasst:
- eine verformbare innere Schicht (108), die bestimmt ist, mit der amputierten unteren Gliedmaße im Kontakt zu sein;
- eine verformbare äußere Schicht (120);
- eine Struktur aus starrem Material (117), die zwischen der inneren Schicht (108) und der äußeren Schicht (120) angeordnet ist, aufweisend mindestens zwei Masten (118), wobei sich jeder Mast (118) etwa axial ab einer Basis (125), die sich auf der distalen Seite befindet, zu einem Ende (126) erstreckt, das sich auf der proximalen Seite befindet,
**dadurch gekennzeichnet, dass**:
- die verformbare innere (108) und äußere (120) Schicht aus Silikongummi sind,
und dass
- die starre Struktur (117) auf die innere Schicht (108) geklebt ist, wobei die innere Schicht (108) zwischen zwei aufeinanderfolgenden Masten (118) im Kontakt mit der äußeren Schicht (120) ist, wobei die Enden (126) der verschiedenen Masten (118) nicht verbunden sind, so dass sie sich unter der Wirkung einer Verformung der inneren Schicht (108) und der äußeren Schicht (120) voneinander abspreizen oder einander annähern.

2. Schaft (100) nach einem der vorangehenden Ansprüche, **dadurch gekennzeichnet, dass** das starre Material Carbon umfasst.

3. Schaft (100) nach einem der vorangehenden Ansprüche, **dadurch gekennzeichnet, dass** die Struktur aus starrem Material (117) vier Masten (118) umfasst.

4. Schaft (100) nach einem der vorangehenden Ansprüche, **dadurch gekennzeichnet, dass** er im Bereich des distalen Teils (106) Befestigungsmittel (116) des modularen Elements (104) aufweist, die mit der Struktur aus starrem Material (117) fest verbunden sind.

5. Verfahren (700) zur Herstellung eines Schafts (100) als Schnittstelle einer amputierten unteren Gliedmaße und eines modularen Elements (104), **dadurch gekennzeichnet, dass** es die folgenden Schritte aufweist:
- Herstellen (701) eines Positivs (101), das die amputierte untere Gliedmaße darstellt, umfassend eine proximale Zone (105) und eine distale Zone (106);
- Aufbringen (704) des Silikongummis auf das Positiv (101), so dass eine Schicht, bezeichnet als innere Schicht (108), bedeckt wird;
- Wärmebehandeln (710) des mit der inneren Schicht (108) bedeckten Positivs (101);
- Aufbringen (712) einer Schicht (114) eines Materials, das imstande ist, versteift zu werden, auf das mit der inneren Schicht (108) bedeckten Positiv (101), so dass es bedeckt ist;
- Versteifen (714) der Schicht (114) aus Material, das imstande ist, versteift zu werden;
- Schneiden (718) der versteiften Schicht (114), so dass eine starre Struktur (117) gebildet wird, die mindestens zwei Masten (118) aufweist, die sich von der distalen Zone (106) zu der proximalen Zone (105) erstrecken;
- Aufbringen (721) des Silikongummis auf das mit der inneren Schicht (108) und der starren Struktur (117) bedeckte Positiv (101), so dass es mit einer Schicht, bezeichnet als äußere Schicht (120), bedeckt ist;
- Wärmebehandeln (723) des mit der inneren Schicht (108), der starren Struktur (117) und der äußeren Schicht (120) bedeckten Positivs (101);
- Entformen (724) der Einheit, die die innere Schicht (108), die starre Struktur (117) und die äußere Schicht (120) aufweist, von dem Positiv (101), wobei der derart entformte Schaft verschiedene Masten (118) aufweist, deren Enden (126) nicht verbunden sind, so dass sie sich unter der Wirkung einer Verformung der inneren Schicht (108) und der äußeren Schicht (120) voneinander abspreizen oder aneinander annähern.

6. Verfahren (700) nach vorangehendem Anspruch, **dadurch gekennzeichnet, dass** der Schritt des Aufbringens (704) von Silikongummi auf das Positiv (101) aufweist:
- Extrudieren (705) des Silikongummis derart, dass mindestens ein Band (112) mit einer etwa konstanten Dicke gebildet wird;
- Bedecken (706) mindestens einer Zone des Positivs (101) durch das Band (112);
- Ausstatten (707) des distalen Teils (106) des Positivs (101);
- Schneiden (708) und/oder Glätten eventueller restlicher Überdicken.

7. Verfahren (700) nach einem der Ansprüche 5 bis 6, **dadurch gekennzeichnet, dass** es zwischen dem Versteifungsschritt (714) und dem Schneideschritt (718) den folgenden Schritt aufweist:
- Entfernen (715) der versteiften Schicht (114) vom Positiv (101), das mit der inneren Schicht (108) bedeckt ist.

8. Verfahren (700) nach vorangehendem Anspruch, **dadurch gekennzeichnet, dass** es nach dem Schneideschritt (718) die folgenden Schritte aufweist:
- Positionieren (719) der starren Struktur (117) auf dem mit der inneren Schicht (108) bedeckten Positiv (101);
- Kleben (720) der starren Struktur (117) auf die innere Schicht (108).

9. Verfahren (700) nach einem der Ansprüche 5 bis 8, **dadurch gekennzeichnet, dass** es nach dem Schritt des Aufbringens (704) der inneren Schicht (108) auf das Positiv (101) die folgenden Schritte aufweist:
- Durchführen (709) einer Vakuumierung des mit der inneren Schicht (108) bedeckten Positivs (101) und des lösbaren Beutels
- Bedecken (711) des mit der inneren Schicht (108) bedeckten Positivs (101) durch einen lösbaren Beutel.

10. Verfahren (700) nach einem der Ansprüche 5 bis 9, **dadurch gekennzeichnet, dass** es nach dem Schritt des Aufbringens (721) der äußeren Schicht (120) auf das Positiv (101) den folgenden Schritt aufweist:
- Durchführen (722) einer Vakuumierung des mit der inneren Schicht (108), der starren Struktur (117) und der äußeren Schicht (120) bedeckten Positivs (101).

11. Verfahren (700) nach einem der Ansprüche 5 bis 10, **dadurch gekennzeichnet, dass** es den folgenden Schritt aufweist:
- Anbringen (716) der Befestigungsmittel (116) an der starren Struktur (117), die zum Zusammenwirken mit dem modularen Element (104) geeignet sind.

## Claims

1. A socket (100) for interfacing an amputated lower limb and a modular element (104), comprising:
- a proximal part (102) allowing the amputated lower limb to be inserted;
- a distal part (103) allowing the modular element (104) to be fastened; the socket also comprising:
- a deformable inner layer (108) for being in contact with the amputated lower limb;
- a deformable outer layer (120);
- a structure of rigid material (117) disposed between the inner layer (108) and the outer layer (120), comprising at least two masts (118), each mast (118) extending substantially axially from a base (125) located on the distal side to an end (126) located on the proximal side,
**characterised in that**:
- the inner (108) and outer (120) deformable layers are made of silicone rubber,
and **in that**
- said rigid structure (117) is adhered to the inner layer (108), the inner layer (108) being in contact with the outer layer (120) between two successive masts (118), the ends (126) of the different masts (118) not being connected so as to move apart from or closer to each other under the effect of a deformation of the inner layer (108) and the outer layer (120).

2. The socket (100) according to one of the preceding claims, **characterised in that** the rigid material comprises carbon.

3. The socket (100) according to one of the preceding claims, **characterised in that** the structure of rigid material (117) has four masts (118).

4. The socket (100) according to one of the preceding claims, **characterised in that** it comprises, at the distal part (106), means for attaching (116) the modular element (104) integral with the structure of rigid material (117).

5. A method (700) for manufacturing a socket (100) for interfacing an amputated lower limb and a modular element (104), **characterised in that** it comprises the following steps of:
- making (701) a positive (101) representing the amputated lower limb, comprising a proximal zone (105) and a distal zone (106);
- applying (704) silicone rubber to the positive (101) so as to cover it with a layer called the inner layer (108);
- stoving (710) the positive (101) covered with the inner layer (108);
- applying (712) a layer (114) of a material capable of being rigidified to the positive (101) covered with the inner layer (108), so as to cover it;
- rigidifying (714) said layer (114) of material capable of being rigidified;
- cutting off (718) said rigidified layer (114) so as to form a rigid structure (117) comprising at least two masts (118) extending from the distal zone (106) to the proximal zone (105);
- applying (721) silicone rubber to the positive (101) covered with the inner layer (108) and the rigid structure (117), so as to cover it with a layer called the outer layer (120);
- stoving (723) the positive (101) covered with the inner layer (108), the rigid structure (117), and the outer layer (120);
- demoulding (724) the assembly comprising the inner layer (108), the rigid structure (117), and the outer layer (120), of the positive (101), the socket thus demoulded comprising different masts (118), the ends (126) of which being not linked so as to move apart from or closer to each other under the effect of a deformation of the inner layer (108) and the outer layer (120).

6. The method (700) according to the preceding claim, **characterised in that** the step of applying (704) silicone rubber to the positive (101) comprises:
- extruding (705) silicone rubber so as to form at least one strip (112) having a substantially constant thickness;
- covering (706) at least one zone of the positive (101) with said strip (112);
- filling out (707) the distal part (106) of the positive (101);
- cutting off (708) and/or smoothing possible residual excess thicknesses.

7. The method (700) according to one of claims 5 to 6, **characterised in that** it comprises, between the rigidifying step (714) and the cutting off step (718) the following step of:
- removing (715) the rigidified layer (114) of the positive (101) covered with the inner layer (108).

8. The method (700) according to the preceding claim, **characterised in that** it comprises, following the cutting step (718), the following steps of:
- positioning (719) the rigid structure (117) on the positive (101) covered with the inner layer (108);
- adhering (720) said rigid structure (117) to the inner layer (108).

9. The method (700) according to one of claims 5 to 8, **characterised in that** it comprises, following the step of applying (704) the inner layer (108) to the positive (101), the following steps of:
- creating a vacuum (709) on the positive (101) covered with the inner layer (108) and the soluble bag (709)
- covering (711) the positive (101) covered with the inner layer (108) with a soluble bag.

10. The method (700) according to one of claims 5 to 9, **characterised in that** it comprises, following the step of applying (721) the outer layer (120) to the positive (101), the following step of:
- creating a vacuum (722) on the positive (101) covered with the inner layer (108), the rigid structure (117), and the outer layer (120).

11. The method (700) according to one of claims 5 to 10, **characterized in that** it comprises the following step of:
- hooking (716) means (116) for attaching to the rigid structure (117), which are adapted to cooperate with the modular element (104).
